# FASCICULE DE BREVET EUROPEEN

(11) **EP 1 458 279 B1**
(45) Date de publication et mention de la délivrance du brevet: **16.01.2008**
(21) Numéro de dépôt: 02801173.2
(22) Date de dépôt: 24.12.2002
(51) Int. Cl.: A61B 5/103

(54) **PROCEDE ET DISPOSITIF D'EVALUATION DES FORCES D'EQUILIBRAGES DU SQUELETTE**
VERFAHREN UND VORRICHTUNG ZUR BESTIMMUNG DER GLEICHGEWICHTSKRÄFTE DES SKELETTS
METHOD AND DEVICE FOR EVALUATING THE SKELETON BALANCE FORCES

(30) Priorité: 28.12.2001 FR 0117126
(43) Date de publication de la demande: 22.09.2004
(73) Titulaire: Warsaw Orthopedic, Inc., Warsaw, IN 46581 (US)
(72) Inventeur: BERTHONNAUD, Eric, F-69260 Charbonnières les Bains (FR); DIMNET. Joannès, F-69300 Caluire Et Cuire (FR); ROUSSOULY, Pierre, F-69450 Saint Cyr au Mont d'Or (FR)
(74) Mandataire: Neyret, Daniel Jean Marie
(86) Numéro de dépôt international: PCT/FR2002/004541
(87) Numéro de publication internationale: WO 2003/055378

(56) Documents cités:
- EP-A- 0 119 660
- FR-A- 2 803 507
- NL-A- 7 415 910

## Description

### DOMAINE TECHNIQUE DE L'INVENTION

La présente invention concerne l'évaluation des forces musculaires et articulaires chez un sujet occupant une posture stable.

On a depuis longtemps analysé l'anatomie des êtres vivants, pour en déterminer notamment le squelette avec ses articulations et les muscles qui commandent ses mouvements.

On connaît en particulier la forme du squelette, les amplitudes de mouvement possibles, les points d'attache des muscles et l'amplitude de leurs mouvements.

Mais on ne sait pratiquement rien sur les valeurs des forces musculaires à l'origine des mouvements.

Et l'on ne sait rien, non plus, sur les valeurs des forces musculaires nécessaires à l'acquisition et au maintien d'une posture stable chez un être vivant, ni sur les forces articulaires induites. Or des forces musculaires et/ou articulaires excessives sont certainement à l'origine de fatigues, de troubles de fonctionnement et de dégradation des articulations, de sorte qu'il y a un intérêt à évaluer ces forces.

Le document US 4 277 828 décrit un système d'analyse pour la détermination de la résultante des forces au niveau d'une articulation osseuse.

### EXPOSE DE L'INVENTION

Le problème proposé par la présente invention est de concevoir un nouveau procédé, et un nouveau dispositif, permettant l'évaluation des forces d'équilibrage du squelette, ou forces musculaires mises en jeu pour l'acquisition et le maintien d'une posture stable, et permettant l'évaluation des forces articulaires qui en résultent.

L'invention résulte de l'observation selon laquelle, pour un être humain occupant une posture stable, chacun des segments corporels constitutifs est supposé être mis en équilibre sur le segment inférieur par l'intermédiaire des forces musculaires qui contrebalancent l'effet de la pesanteur. Comme dans l'analyse des mouvements relatifs entre les segments, on considère les segments supposés indéformables : tête, avant-bras, bras, pieds, jambes, cuisses. Contrairement à l'analyse des mouvements, dans laquelle le tronc est considéré comme un seul ensemble pratiquement rigide, l'invention admet que le tronc est constitué d'un segment cervical correspondant à la colonne cervicale, d'un segment dorsal correspondant à la colonne dorsale, d'un segment lombaire correspondant à la colonne lombaire, et du bassin. L'invention retient la constatation selon laquelle une posture stable n'est jamais rigide ; des corrections instantanées de posture existent, qui ne modifient pas les courbures cervicales, dorsales ou lombaires, mais font intervenir leurs positions angulaires relatives.

Pour l'évaluation des forces musculaires et articulaires, l'invention part de deux informations préalables.

La première information concerne la forme et la position relative des segments corporels équilibrés. La morphologie des segments corporels constitutifs et leurs positions relatives sont obtenues par l'intermédiaire d'un cliché radiographique. On détermine ainsi les extrémités articulées de chaque segment de squelette, et leurs positions relatives.

La seconde information préalable concerne les forces pesantes appliquées sur la structure corporelle et qui tendent à la déséquilibrer. Cette information est recherchée sous la forme de l'axe global de gravité, qui passe par le centre de gravité globale du sujet en posture équilibrée, et dont la position est généralement déportée par rapport au squelette.

Dans une posture globale, on considère ainsi que la tête constitue le segment terminal, qui est tout d'abord mis en équilibre par rapport au segment inférieur constitué par le segment cervical. L'ensemble de la tête et du segment cervical est équilibré sur le segment suivant constitué par le segment dorsal. De proche en proche, toutes les articulations entre groupes de segments supérieurs et inférieurs sont équilibrées par une mise en équilibre décroissante. A la fin, l'ensemble du corps entier est mis en équilibre autour de la cheville.

Ainsi, pour évaluer les forces musculaires et articulaires d'équilibrage du squelette d'un sujet, l'invention prévoit un procédé comprenant les étapes suivantes :
a) réaliser une image radiographique du squelette à étudier, prise dans un plan généralement perpendiculaire aux axes de rotation des articulations du squelette à étudier,
b) déterminer simultanément la position de l'axe global de gravité du corps sur l'image radiographique,
c) sélectionner, sur l'image radiographique, les segments de squelette articulés les uns aux autres, repérer leurs positions et celles des articulations et affecter à chaque segment le poids de segment corporel correspondant,
d) repérer sur l'image radiographique les directions et points d'ancrage des principaux muscles tenseurs d'équilibrage du squelette à étudier, .
e) déterminer par calcul l'intensité de la force de rappel exercée par les principaux muscles tenseurs en considérant qu'elle compense le moment de rotation de la force gravitaire appliqué à chaque segment autour de l'articulation inférieur dudit segment.

De préférence, le procédé détermine en outre par calcul la force d'appui de chaque segment sur son articulation inférieure, en considérant qu'elle est égale à la résultante de la force gravitaire et de la force de rappel appliquée sur le segment.

En pratique, pour la mise en oeuvre d'un tel procédé d'évaluation, on procède de la façon suivante :
- on sélectionne comme segments articulés tout ou partie des segments suivants : la tête, le segment cervical, le segment dorsal, le segment lombaire, le segment de bassin, les fémurs,
- pour l'équilibrage, on considère comme principaux muscles tenseurs d'équilibrage : les principaux muscles agoniste, antagoniste, latéral et médian. Ces muscles recrutés pour participer à l'équilibrage sont choisis par l'utilisateur clinicien en fonction de la posture d'ensemble rachis bassin.

On peut avantageusement tenir compte des légers mouvements du sujet lorsqu'on le place en une position d'équilibre stable. Pour cela, on mémorise les positions successives de l'axe global de gravité pendant quelques secondes avec une fréquence de plusieurs acquisitions par seconde, l'ensemble des positions mémorisées constituant une nuée elliptique de positions. On détermine l'ellipse de dispersion qui enveloppe ladite nuée de positions, et on sélectionne les images radiographiques correspondant à un axe global de gravité dont la position est comprise dans l'ellipse de dispersion.

On peut aussi déterminer par calcul, en fonction de l'ellipse de dispersion, l'incertitude dans l'évaluation des forces de rappel ou d'appui.

L'invention prévoit également un dispositif pour l'évaluation des forces d'équilibrage du squelette, comprenant :
- une source de rayons X et des moyens supports de plaque cible sensible aux rayons X,
- un plateau support de sujet, adapté pour supporter le sujet en position fixe entre la source de rayons X et les moyens supports de plaque cible et pour générer des signaux de position images de la position horizontale de l'axe global de gravité du sujet,
- des moyens pour numériser l'image radiographique du sujet sur la plaque cible, et pour générer ainsi une image radiographique numérisée mémorisée dans une mémoire appropriée,
- des moyens pour superposer sur l'image radiographique numérisée du sujet l'image numérisée de l'ombre portée de l'axe global de gravité sur la plaque cible,
- des moyens pour sélectionner, sur l'image radiographique numérisée, les segments de squelette articulés et leurs articulations, ainsi que les points d'ancrage des muscles tenseurs d'équilibrage,
- des moyens de calcul et un programme pour calculer l'intensité de la force de rappel exercée par les muscles tenseurs d'équilibrage en considérant qu'elle compense le moment de rotation de la force gravitaire appliquée à chaque segment autour de l'articulation inférieure dudit segment.

De préférence, le dispositif comprend en outre des moyens pour évaluer les variations dans la posture verticale du sujet par les dimensions de l'ellipse de dispersion de position horizontale de son axe global de gravité lorsqu'il occupe une position stable.

Selon un mode de réalisation avantageux, le programme comprend une ou plusieurs séquences d'acquisition selon lesquelles :
- l'opérateur pointe sur l'image radiographique les positions des articulations, des segments et des points d'ancrage des muscles tenseurs d'équilibrage,
- les moyens pour numériser l'image radiographique du sujet mémorisent dans la mémoire les positions pointées par l'opérateur.

### DESCRIPTION SOMMAIRE DES DESSINS

D'autres objets, caractéristiques et avantages de la présente invention ressortiront de la description suivante de modes de réalisation particuliers, faite en relation avec les figures jointes, parmi lesquelles:
- la figure 1 est une vue de côté illustrant les principaux segments corporels du corps humain, avec leurs articulations et la position de l'axe global de gravité du corps ;
- la figure 2 est une vue de côté illustrant l'équilibrage partiel par les forces de pesanteur, musculaires et de contact entre un sous-ensemble supérieur constitué par la tête et le segment cervical et le segment corporel adjacent inférieur ;
- la figure 3 illustre le calcul des forces dans l'équilibrage du sous-ensemble supérieur constitué par la tête et le segment cervical ;
- la figure 4 est une vue de côté illustrant les positions et directions des muscles principaux assurant l'équilibrage global de la posture debout du corps humain ;
- la figure 5 est une vue de côté illustrant la détermination de la résultante des forces musculaires dans la zone du genou ;
- la figure 6 est une vue de côté illustrant le schéma de principe de détermination des forces d'équilibrage dans la zone du genou ;
- la figure 7 est une vue schématique en perspective d'un dispositif selon l'invention pour la prise de clichés radiographiques avec détermination de la position de l'axe global de gravité ; et

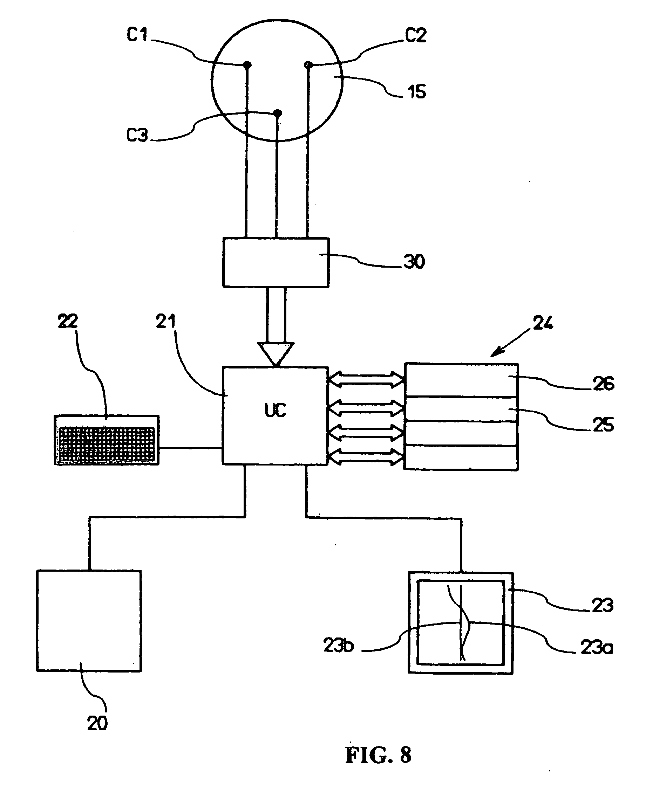

- la figure 8 est un schéma théorique illustrant les moyens essentiels de calcul pour l'évaluation des forces d'équilibrage selon l'invention.

### DESCRIPTION DES MODES DE REALISATION PREFERES

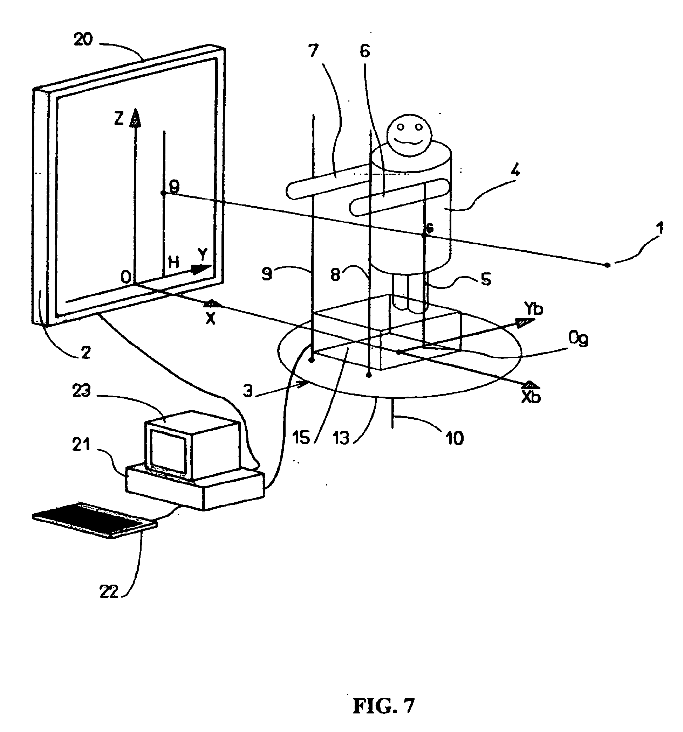

On considère tout d'abord le dispositif illustré sur la figure 7, permettant par exemple l'acquisition des données pour l'analyse de la posture debout d'un sujet 4.

Le dispositif comprend une source de rayons X 1, des moyens supports de plaque cible 2 pour supporter une plaque cible sensible aux rayons X et orientée verticalement à une certaine distance horizontale de la source de rayons X 1, et des moyens supports de sujet 3 adaptés pour supporter un sujet 4 en position fixe entre la source de rayons X 1 et les moyens supports de plaque cible 2. Le dispositif est ainsi une installation pour la prise de radiographies. La source de rayons X 1 et les moyens supports de plaque cible 2 peuvent avoir une structure telle que celles habituellement utilisées dans les installations de radiographies médicales.

Dans le dispositif de la figure 7, les moyens supports de sujet 3 comprennent des moyens pour détecter la position horizontale du plan support et pour repérer dans ce plan la position instantanée de l'axe global de gravité 5 du sujet 4 ou axe vertical passant par le centre de gravité G du sujet 4, lors du fonctionnement de la source de rayons X 1. L'axe global de gravité 5 instantané est saisi lors de la prise du cliché radiographique, et sa position est projetée dans le plan de l'image radiographique à partir de la source de rayons X 1 suivant une projection conique. Un principe de capteur de position de l'axe global de gravité et de projection sur l'image radiographique est décrit dans le document WO 01/50956 ou son équivalent FR 2 803 507 A.

Par exemple, les moyens supports de sujet 3 comprennent un plateau de force de balance modifiée 15 muni d'une pluralité de capteurs de force répartis horizontalement selon des positions repérés par rapport à un repère Xb Yb, et associés à des moyens de calcul 21 pour calculer la position Og, dans le plan horizontal, de la résultante des forces appliquées par le sujet 4 sur le plateau de force 15 dans le repère Xb Yb. Les moyens de calcul 21 peuvent par exemple être l'unité de calcul d'un micro-ordinateur ayant également un écran de visualisation 23 et un clavier 22.

En position debout, les bras 6 et 7 du sujet 4 peuvent être laissés libres. En alternative, on peut trouver intérêt à prévoir des supports 8 et .9 sur lesquels le sujet peut poser ses mains ou ses bras, permettant de déterminer une position stable et reproductible du sujet 4.

Dans le dispositif de la figure 7, on prévoit en outre des moyens 20 pour numériser par balayage l'image radiographique du sujet sur les moyens supports de plaque cible 2. On peut pour cela utiliser un scanner adapté pour scruter par balayage l'image radiographique réalisée sur la plaque cible 2 sensible aux rayons X et pour produire une suite de signaux numériques constituant l'image radiographique numérisée. Cette image est envoyée aux moyens de calcul 21, qui en effectuent la mémorisation et le traitement.

Le traitement comprend en particulier une opération consistant à superposer sur l'image radiographique du sujet l'image de l'ombre portée Hg de l'axe global de gravité 5 du sujet sur la plaque cible 2 contenant l'image radiographique. Cette superposition peut se faire par les moyens de calcul 21, qui reçoivent du plateau de force 15 l'information des coordonnées de la projection horizontale Og du centre du gravité G, qui en déduisent la position de l'ombre portée Hg et qui l'introduisent dans l'image numérisée contenue en mémoire.

Un tel dispositif peut être tel que celui décrit dans le document WO 01/50956, auquel on pourra se référer pour plus de détails.

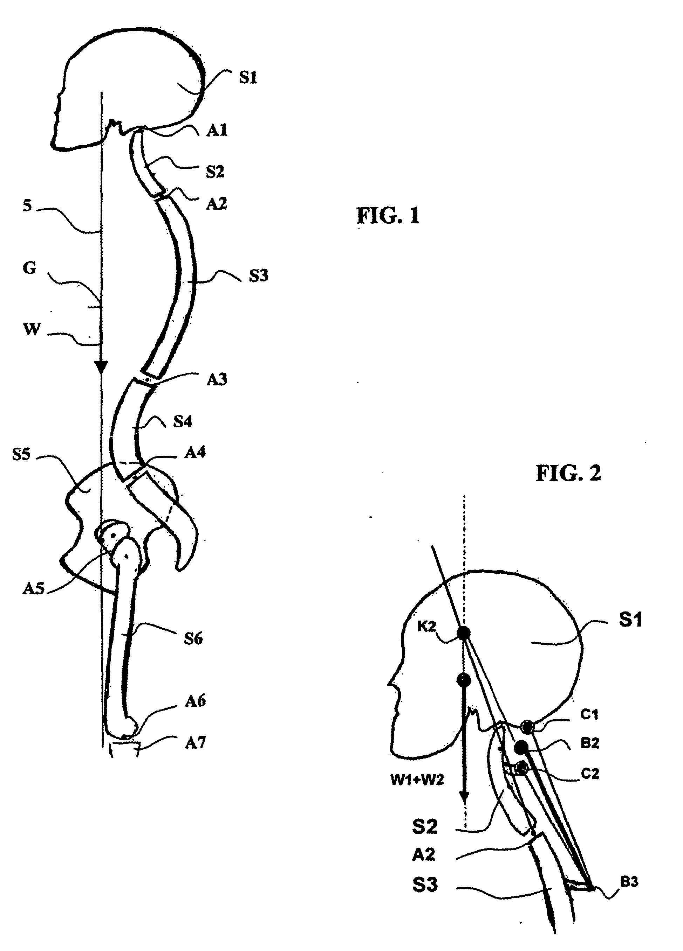

On considérera maintenant les figures 1 à 4, pour mieux comprendre le procédé d'évaluation de forces selon l'invention, et le fonctionnement du dispositif de la figure 7.

La figure 1 schématise la forme des principaux segments du squelette humain, leurs positions relatives et les points d'articulation. On distingue ainsi le segment supérieur S1 formé par la tête, le second segment S2 formé par la colonne cervicale, le troisième segment S3 formé par la colonne dorsale, le quatrième segment S4 formé par la colonne lombaire, le cinquième segment S5 formé par le bassin, un sixième segment S6 formé par le fémur, et la partie supérieure d'un septième segment S7 formé par le tibia. on distingue les articulations intermédiaires respectives A1, A2, A3, A4, A5 et A6 telles qu'illustrées sur la figure.

A chaque segment S1-S7 du squelette correspond un segment du corps, comprenant le squelette et les autres organes situés dans la même zone de hauteur du corps. Généralement, les autres organes sont déportés par rapport au squelette.

On part de l'hypothèse que le centre de gravité de chacun des segments du corps est aligné sur la verticale passant par le centre de gravité G de l'ensemble. Cette verticale est représentée par l'axe global de gravité 5 tel que déterminé par le dispositif de la figure 7 comme indiqué précédemment.

Le segment de tête S1 est articulé sur le point d'articulation A1. L'ensemble du segment de tête S1 et du segment cervical S2 est articulé sur le point d'articulation A2. L'ensemble du segment de tête S1, du segment cervical S2 et du segment dorsal S3 est articulé sur le point d'articulation A3. L'ensemble formé par le segment de tête S1, le segment cervical S2, le segment dorsal S3 et le segment lombaire S4 est articulé sur le point d'articulation A4, et ainsi de suite.

Chaque segment de corps a un poids propre. Des études anatomiques publiées précédemment définissent chaque poids segmentaire comme une proportion du poids total du corps.

On considère maintenant, à titre d'exemple, le modèle de l'équilibrage des deux segments supérieurs S1 et S2 sur le troisième segment S3, tel qu'illustré sur la figure 2. L'ensemble S1 et S2 est susceptible de tourner autour de l'articulation A2 du segment dorsal S3, dans le plan de la figure. L'ensemble S1 et S2 est soumis à une force de pesanteur W1 + W2 dont la valeur relative par rapport au poids total W est connue d'après les travaux antérieurs sur l'anatomie. Cette force de pesanteur W1 + W2 est appliquée en un centre de gravité local porté par la direction connue de l'axe global de gravité 5.

Cette force de pesanteur W1 + W2 sollicite l'ensemble S1 et S2 en rotation autour du point d'articulation A2 identifié sur le cliché radiographique, et donc identifié sur l'image radiographique numérisée. Son action rotatoire est compensée par la force exercée par les muscles. Dans l'exemple proposé, deux faisceaux musculaires interviennent : l'un s'insère sur le segment S3 au niveau de la troisième vertèbre dorsale, l'insertion étant désignée par la référence B3 sur la figure 2, et s'insère sur la tête S1 au point C1 ; un autre faisceau va de la même insertion dorsale B3 à la quatrième vertèbre cervicale sur laquelle il s'insère au point C2. L'action résultante des muscles combine l'action des deux faisceaux, et l'on considère que la force résultante des muscles suit une direction moyenne B3B2 ainsi déterminée à partir du cliché radiographique.

On se trouve ainsi conduit à résoudre un problème de mécanique statique plane faisant intervenir trois forces :
- une force de pesanteur dont la direction verticale est connue, c'est-à-dire la direction de l'axe global de gravité, et dont la valeur est connue et vaut W1 + W2, obtenue à partir de tableaux de valeurs connues ;
- une force musculaire dont la direction B3B2 est définie à partir du cliché radiographique ou de l'image radiographique numérisée, et dont la grandeur est inconnue ;
- une force de contact passant par l'articulation A2, et dont la grandeur et la direction sont inconnues.

Ce problème est résolu graphiquement, soit directement sur le cliché radiographique, soit de préférence par calcul vectoriel en utilisant les données de l'image radiographique numérisée.

Pour connaître la valeur de la force d'équilibrage exercée par les muscles, un calcul simple consiste à considérer que la force d'équilibrage compense le moment de rotation de la force de pesanteur appliquée à l'ensemble, autour de l'articulation inférieure A2. On connaît en effet la direction des deux forces, ainsi que l'intensité de la force de pesanteur, et l'on en déduit l'intensité de la force musculaire.

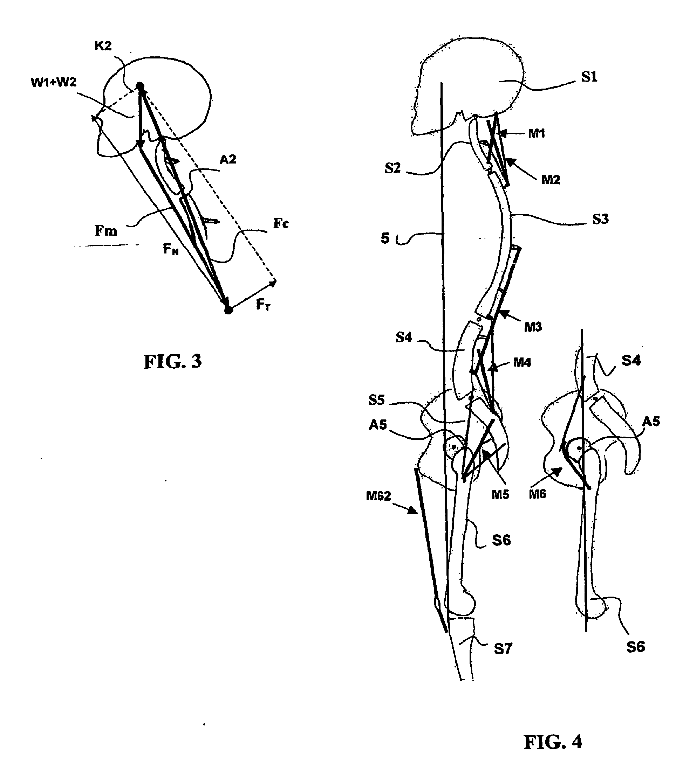

Une méthode globale de résolution, telle qu'illustrée sur la figure 3, permet de résoudre le problème et de connaître simultanément la force musculaire et la force de contact. Pour cela, on considère que la direction de la force musculaire résultante B3B2 rencontre l'axe global de gravité 5 au point K2. Ce point K2 définit également la direction de la force de contact Fc, puisque de cette manière le moment rotatoire des trois forces est nul autour de l'articulation A2.

On écrit ensuite que la résultante géométrique de la force de gravité W1 + W2 portée par l'axe global de gravité 5, de la force musculaire Fm portée par la direction B3B2, et de la force de contact Fc de direction A2K2 connue est nulle. Ce calcul est visualisé sur la figure 3, où l'on peut tracer Fc dans la direction A2K2, puis W1 + W2 dans la direction verticale à partir de K2, et on en déduit Fm reliant les extrémités des deux vecteurs précédents. La même détermination de la force Fm se fait par calcul sur les données de l'image radiographique numérisée. On obtient ainsi la grandeur de la force musculaire Fm, et la grandeur de la force de contact Fc. Cette dernière peut être décomposée en une composante normale FN perpendiculaire à la zone de contact dans l'articulation A2, et une composante tangentielle FT parallèle à la zone de contact A2. FN représente la force pressante due à l'ensemble S1 et S2 sur S3. FT représente la force de cisaillement dans la zone de contact A2.

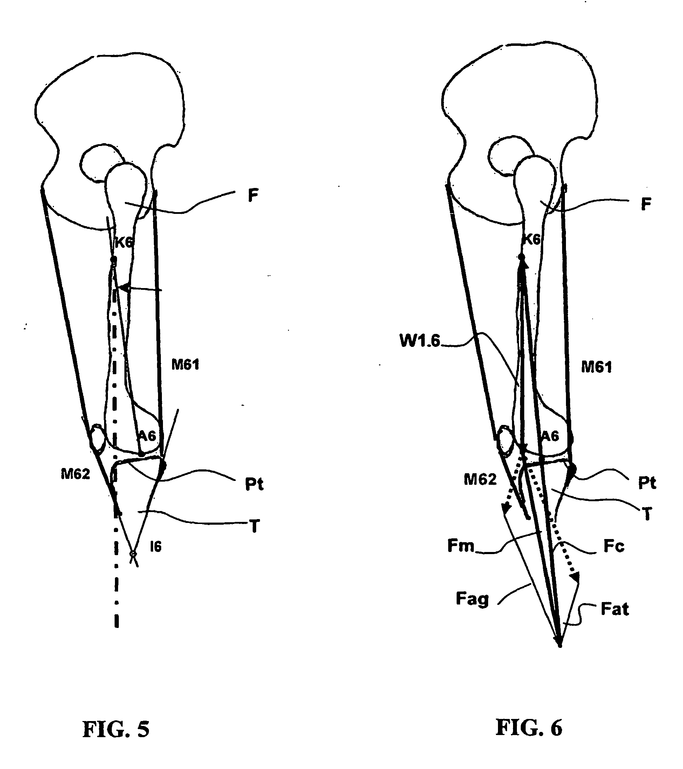

Parmi l'ensemble des muscles participant à l'équilibrage de la posture, l'invention choisit de considérer les muscles qui jouent un rôle primordial. Ces muscles sont illustrés sur la figure 4.

Ainsi, pour l'équilibrage de la tête S1, on considère le muscle M1 constitué par le grand complexus.

Pour l'équilibrage du segment cervical S2, on considère la partie cervicale des muscles de la nuque, tels que le latissimus dorsi et le trapèze M2.

Pour l'équilibrage du segment dorsal S3, on considère le muscle M3 constitué par le grand dorsal.

Pour l'équilibrage du segment lombaire S4, on considère les gouttières lombaires M4 dont les faisceaux vont de la vertèbre dorsale D12 à la vertèbre lombaire L3.

Pour l'équilibrage du bassin S5, on considère le muscle M5 constitué par le grand fessier qui intervient lorsque l'axe global de gravité passe en avant du centre des têtes fémorales ; ce muscle s'insère en éventail le long de la vertèbre sacrée ; lorsque l'axe global de gravité passe en arrière du centre des têtes fémorales, on considère le muscle M6 constitué par le psoas, qui contourne la tête fémorale.

Pour l'équilibrage des fémurs, on prend en compte le muscle droit antérieur M62 et les ischios-jambiers M61 (voir figure 6).

A titre de second exemple, on a illustré sur les figures 5 et 6 l'évaluation des forces d'équilibrage de l'articulation du genou.

Le genou est l'articulation entre le fémur F et le tibia T. Elle présente une particularité anatomique. Le fémur F peut tourner, mais aussi se déplacer le long du plateau tibial Pt, de sorte qu'il tourne et glisse. Ce glissement est mis en évidence cliniquement par le test du tiroir.

Selon l'invention, on considère que le contact entre fémur F et tibia T se fait sans frottement pour respecter la condition de roulement et de glissement. Les forces musculaires agonistes (dans le sens du mouvement imposé par la pesanteur) et antagonistes (dans le sens contraire) sont telles qu'elles sont compatibles avec un contact sans frottement.

La force de pesanteur W1-6 des segments situés au-dessus du genou est connue en grandeur et direction. Elle est la somme des forces de pesanteur W1, W2, W3, W4, W5 et W6. Cette force W1-6 est portée par l'axe global de gravité.

La direction de la force de contact Fc est connue : elle passe par le point A6 de contact entre le fémur F et le tibia T, identifiable sur la radiographie. Elle est perpendiculaire au plateau tibial Pt également visible sur la radiographie. Le muscle droit antérieur M62 produit une force agoniste Fag, tandis que les ischios-jambiers M61 produisent une force antagoniste Fat, dont les directions d'action sur le tibia T sont illustrées sur la figure 5 et se recoupent au point 16.

Pour la détermination des forces, on procède comme précédemment, comme indiqué maintenant en relation avec les figures 5 et 6. La direction de la force de contact Fc, qui passe par le point de contact A6, recoupe l'axe global de gravité 5 au point K6. La résultante Fm des forces musculaires agoniste et antagoniste doit passer par K6 pour annuler les effets rotatoires de la pesanteur. Cette résultante passe aussi par le point 16. Ainsi; la direction de la résultante des efforts musculaires est I6K6.

Comme illustré sur la figure 6, on peut résoudre le problème par un tracé des vecteurs en appliquant la règle de fermeture du triangle des forces pour un système en équilibre. Connaissant la force de pesanteur W1-6, et connaissant la direction de la force de contact Fc, on en déduit la force musculaire Fm que l'on décompose ensuite pour trouver la force agoniste Fag et la force antagoniste Fat. Le même calcul peut être effectué sur l'image radiographique numérisée.

On se réfère maintenant à nouveau à la figure 7 illustrant le dispositif selon un mode de réalisation de l'invention.

Après avoir pris l'image radiographique et repéré la position de l'axe global de gravité 5, l'opérateur procède à l'évaluation des forces musculaires et de pression sur les articulations.

Selon une première possibilité, il considère l'image radiographique telle que présente sur la plaque sensible 2 dans les moyens supports, et il pointe sur cette image radiographique les positions des articulations, des segments et des points d'ancrage des muscles tenseurs d'équilibrage. Les moyens 20 pour numériser l'image radiographique du sujet mémorisent simultanément les points désignés par l'opérateur, qui sont ensuite utilisés pour les calculs de forces.

Selon une autre possibilité, l'opérateur visualise sur un écran 23 l'image radiographique numérisée ainsi que la position de l'axe global de gravité 5, et pointe à l'aide du clavier 22 ou d'une souris les positions des articulations, des segments et des points d'ancrage des muscles tenseurs d'équilibrage. Les moyens de calcul 21 et le programme associé permettent de mémoriser les points désignés par l'opérateur, pour l'exécution ultérieure des calculs de forces.

Un programme d'acquisition spécifique permet à l'utilisateur d'acquérir un point anatomique à la souris, sur l'image numérisée du film. Des techniques issues du traitement d'image sont incorporées au programme pour faciliter l'acquisition d'un point ou d'un détail. Ce sont : un effet de grossissement, une augmentation des contrastes pour rendre utilisable un cliché flou, des aides pour acquérir des tangentes à des profils osseux, ou pour identifier de façon reproductible le centre de contours quasi circulaires.

Un programme de représentation numérique permet à l'utilisateur de visualiser les différentes démarches du calcul des forces musculaires et de contact, à partir de la forme de la posture.

Le programme contenu dans les moyens de calcul 21 permet ensuite d'effectuer les calculs de forces selon le principe indiqué plus haut, et le programme visualise les résultats sur l'écran 23 ou sur tout autre support approprié.

De préférence, le programme prend en compte la dimension de l'ellipse de dispersion de position horizontale de l'axe global de gravité pour calculer une marge d'incertitude du calcul des forces musculaires et de contact.

Ainsi, selon l'invention, en exploitant un nombre réduit de points repérés sur les radiographies, correspondant aux zones d'articulation et aux directions des actions musculaires équilibrantes, et en utilisant la position de l'axe global de gravité du sujet, l'invention permet de calculer la valeur de la force musculaire principale assurant l'équilibre d'un segment ou d'un ensemble de segments préalablement équilibrés par rapport aux segments suivants. Les grandeurs et directions des forces de contact induites au niveau de l'articulation sont également déterminées.

Ces évaluations mettent en oeuvre des lois simples de la statique, et des connaissances en physiologie des articulations.

L'invention permet ainsi l'évaluation des forces musculaires et des forces de pression sur les articulations, sans nécessiter l'implantation de capteurs ou autres instruments de mesure à l'intérieur du corps humain.

L'invention peut trouver application dans l'évaluation a priori des forces, pour l'étude d'une posture donnée.

L'invention trouve également application dans la comparaison de plusieurs évaluations successives de forces d'un même sujet, par exemple avant et après opération, ou avant et après traitement orthopédique. Il est ainsi possible de montrer des variations beaucoup plus significatives que celles qui traduisent les changements de morphologie.

La présente invention n'est pas limitée aux modes de réalisation qui ont été explicitement décrits, mais elle en inclut les diverses variantes et généralisations contenues dans le domaine des revendications ci-après.

## Revendications

1. - Procédé d'évaluation des forces d'équilibrage du squelette d'un sujet, comprenant les étapes suivantes :
a) réaliser une image radiographique du squelette à étudier, prise dans un plan généralement perpendiculaire aux axes de rotation des articulations du squelette à étudier,
b) déterminer simultanément la position de l'axe global de gravité (5) du corps sur l'image radiographique,
c) sélectionner, sur l'image radiographique, les segments de squelette articulés les uns aux autres, repérer leurs positions et celles des articulations et affecter à chaque segment le poids de segment corporel correspondant,
d) repérer sur l'image radiographique les directions et points d'ancrage des principaux muscles tenseurs d'équilibrage du squelette à étudier,
e) déterminer par calcul l'intensité de la force de rappel exercée par les principaux muscles tenseurs en considérant qu'elle compense le moment de rotation de la force gravitaire appliqué à chaque segment autour de l'articulation inférieur dudit segment.

2. - Procédé selon la revendication 1, **caractérisé en ce qu'**on détermine en outre par calcul la force d'appui de chaque segment sur son articulation inférieure, en considérant qu'elle est égale à la résultante de la force gravitaire et de la force de rappel appliquée sur le segment.

3. - Procédé selon l'une des revendications 1 ou 2, **caractérisé en ce que** :
- on sélectionne comme segments articulés tout ou partie des segments suivants : la tête (S1), le segment cervical (S2), le segment dorsal (S3), le segment lombaire (S4), le segment de bassin (S5), les fémurs (S6),
- pour l'équilibrage, on considère comme principaux muscles tenseurs d'équilibrage : les principaux muscles agoniste, antagoniste, latéral et médian. Ces muscles recrutés pour participer à l'équilibrage sont choisis par l'utilisateur clinicien en fonction de la posture d'ensemble rachis bassin.

4. - Procédé selon l'une quelconque des revendications 1 à 3, **caractérisé en ce qu'**on place le sujet en une position d'équilibre stable, on mémorise les positions successives de l'axe global de gravité (5) pendant quelques secondes avec une fréquence de plusieurs acquisitions par seconde, l'ensemble des positions mémorisées constituant une nuée elliptique de positions, on détermine l'ellipse de dispersion qui enveloppe ladite nuée de positions, et on sélectionne les images radiographiques correspondant à un axe global de gravité (5) dont la position est comprise dans l'ellipse de dispersion.

5. - Procédé selon la revendication 4, **caractérisé en ce qu'**on détermine par calcul, en fonction de l'ellipse de dispersion, l'incertitude dans l'évaluation des forces de rappel ou d'appui.

6. - Dispositif pour l'évaluation des forces d'équilibrage du squelette, comprenant :
- une source de rayons X (1) et des moyens supports de plaque cible (2) sensible aux rayons X,
- un plateau support de sujet (3), adapté pour supporter le sujet (4) en position fixe entre la source de rayons X et les moyens supports de plaque cible (2) et pour générer des signaux de position image de la position horizontale (Og) de l'axe global de gravité (5) du sujet (4),
- des moyens (20) pour numériser l'image radiographique du sujet sur la plaque cible, et pour générer ainsi une image radiographique numérisée mémorisée dans une mémoire appropriée,
- des moyens (21) pour superposer sur l'image radiographique numérisée du sujet l'image numérisée de l'ombre portée (Hg) de l'axe global de gravité (5) sur la plaque cible (2),
- des moyens (22) pour sélectionner, sur l'image radiographique numérisée, les segments de squelette articulés et leurs articulations, ainsi que les points d'ancrage des muscles tenseurs d'équilibrage,
- des moyens de calcul (21) et un programme pour calculer l'intensité de la force de rappel exercée par les muscles tenseurs d'équilibrage en considérant qu'elle compense le moment de rotation de la force gravitaire appliquée à chaque segment autour de l'articulation inférieure dudit segment.

7. - Dispositif selon la revendication 6, **caractérisé en ce qu'**il comprend des moyens pour évaluer les variations dans la posture verticale du sujet par les dimensions de l'ellipse de dispersion de position horizontale de son axe global de gravité (5) lorsqu'il occupe une position stable.

8. - Dispositif selon l'une des revendications 6 ou 7, **caractérisé en ce que** le programme comprend une ou plusieurs séquences d'acquisition selon lesquelles :
- l'opérateur pointe sur l'image radiographique les positions des articulations des segments et des points d'ancrage des muscles tenseurs d'équilibrage,
- les moyens (20) pour numériser l'image radiographique du sujet mémorisent dans la mémoire les positions pointées par l'opérateur.

9. - Dispositif selon l'une des revendications 6 ou 7, **caractérisé en ce que** le programme comprend une ou plusieurs séquences d'acquisition selon lesquelles:
- l'opérateur visualise sur un écran (23) l'image radiographique numérisée,
- l'opérateur pointe sur ladite image radiographique numérisée, au moyen d'un dispositif d'entrée -sortie tel qu'un clavier (22) ou une souris, les positions des articulations, des segments et des points d'ancrage des muscles tenseurs d'équilibrage,
- et le programme mémorise les coordonnées des points désignés par l'opérateur.

10. - Dispositif selon l'une des revendications 8 ou 9, **caractérisé en ce que** le programme prend en compte la dimension de l'ellipse de dispersion de position horizontale de l'axe global de gravité (5) pour calculer une marge d'incertitude du calcul des forces musculaires et de contact.

## Claims

1. Method of evaluating balance forces on the skeleton of a subject, comprising the following steps:
a) producing a radiographic image of the skeleton to be studied, taken in a plane generally perpendicular to the axes of rotation of the joints of the skeleton to be studied,
b) determining simultaneously the position of the global axis of gravity (5) of the body on the radiographic image,
c) selecting, on the radiographic image, all of the articulating segments of the skeleton, locating their positions and those of the joints and assigning to each segment the weight of the corresponding segment of the body,
d) locating on the radiographic image the directions and the anchorage points of the principal tensor balance muscles of the skeleton to be studied,
e) determining by calculation the strength of the compensating force exerted by the principal tensor muscles, by considering that it compensates for the moment of rotation of the force of gravity applied to each segment about the lower joint of the said segment.

2. Method according to claim 1, **characterised in that** in addition the support force of each segment on its lower joint is determined by calculation, by considering that it is equal to the resultant of the force of gravity and the compensating force applied to the segment.

3. Method according to one of claims 1 or 2, **characterised in that**:
as articulated segments are selected all or some of the following segments: the head (S1), the cervical segment (S2), the dorsal segment (S3), the lumbar segment (S4), the pelvic segment (S5), the femurs (S6),
for balancing, the principal tensor balance muscles are considered:
the principal muscles of extension, deflection; lateral and median. These muscles, chosen to participate in balance, are picked by the clinical user as a function of the posture of the whole pelvic rachis.

4. Method according to any one of claims 1 to 3, **characterised in that** the subject is placed in a position of stable balance, the successive positions of the global axis of gravity (5) are stored during a few seconds, with a frequency of several acquisitions per second, the aggregate of the stored positions forming an elliptical cloud of positions, the ellipse of dispersion which encompasses the said cloud of positions is determined, and the radiographic images are selected corresponding to a global axis of gravity (5) of which the position is located in the ellipse of dispersion.

5. Method according to claim 4, **characterised in that** the error in the evaluation of the compensating and support forces is determined by calculation, as a function of the ellipse of dispersion.

6. Apparatus for evaluating the balance forces on a skeleton, comprising:
a source of X-rays (1) and support means for a target plate (2) sensitive to X-rays,
a support plate (3) for the subject, adapted to support the subject (4) in a fixed position between the source of the X-rays and the support means of the target plate (2) and for generating signals of the image position of the horizontal position (Og) of the global axis of gravity (5) of the subject (4),
means (20) for digitising the radiographic image of the subject on the target plate, and for generating thus a stored digital radiographic image in an appropriate memory,
means (21) for superimposing on the digital radiographic image of the subject a digital image of the shadow (Hg) of the global axis of gravity (5) projected on the target plate (2),
means (22) for selecting, on the digital radiographic image, the articulated segments of the skeleton and their joints, as well as the anchorage points of the tensor balance muscles,
calculation means (21) and a program for calculating the strength of the compensating force exerted by the tensor balance muscles by considering that it compensates the moment of rotation of the force of gravity applied to each segment about the lower joint of the said segment.

7. Apparatus according to claim 6, **characterised in that** it comprises means for evaluating the variations in the vertical posture of the subject by the dimensions of the ellipse of dispersion of the horizontal position of its global axis of gravity (5) when it occupies a stable position.

8. Apparatus according to one of claims 6 or 7, **characterised in that** the program includes one or more acquisition sequences according to which:
the operator registers on the radiographic image the position of the joints of the segments and of the anchorage points of the tensor balance muscles,
the means (20) for digitising the radiographic image of the subject stores in the memory the positions registered by the operator,

9. Apparatus according to one of claims 6 or 7, **characterised in that** the program comprises one or more acquisition sequences according to which:
the operator displays on a screen (23) the digital radiographic image,
the operator registers on the said digital radiographic image, by means of an input/output device such as a keyboard (22) or a mouse, the positions of the joints, the segments and the anchorage points of the tensor balance muscles,
and the program stores the coordinates of the points indicated by the operator.

10. Apparatus according to one of claims 8 or 9, **characterised in that** the program takes into account the dimension of the ellipse of dispersion of the horizontal position of the global axis of gravity (5) to calculate a margin of error of the calculation of the muscular forces and the contact.

## Patentansprüche

1. Verfahren zur Bewertung der Gleichgewichtskräfte des Skelettes eines Individuums, folgende Schritte umfassend:
a) Aufnahme eines Röntgenbildes des zu untersuchenden Skelettes, aufgenommen in einer im Allgemeinen rechtwinklig zu den Rotationsachsen der Gelenke des zu untersuchenden Skelettes stehenden Ebene,
b) gleichzeitig Bestimmung der Stellung der globalen Schwereachse (5) des Körpers auf dem Röntgenbild,
c) Auswahl der aneinander angelenkten Segmente des Skelettes auf dem Röntgenbild, Bestimmung ihrer Positionen und derer der Gelenke und Zuordnung des Gewichtes des entsprechenden Körpersegmentes zu jedem Segment.
d) Bestimmung der Angriffsrichtungen und -punkte der hauptsächlichen Gleichgewichts-Spannmuskein des zu untersuchenden Skelettes auf dem Röntgenbild,
e) Bestimmung der Größe der Rückstellkraft, die von den hauptsächlichen Spannmuskeln ausgeübt wird, durch Berechnung, aus der Tatsache, dass sie das Drehmoment der Schwerkraft, die an jedes Segment angreift, um das untere Gelenk des genannten Segmentes kompensiert.

2. Verfahren nach Patentanspruch 1, **dadurch gekennzeichnet, dass** außerdem durch Berechnung die Stützkraft jedes Segmentes auf sein unteres Gelenk aus der Tatsache bestimmt wird, dass sie der Resultante aus der Schwerkraft und der Rückstellkraft, die an das Segment angreifen, gleich ist.

3. Verfahren nach einem der Patentansprüche 1 oder 2, **dadurch gekennzeichnet, dass**
- als angelenkte Segmente alle oder ein Teil der folgenden Segmente gewählt werden; der Kopf (S1), das Halswirbel-Segment (S2), das Rückenwirbel-Segment (S3), das Kreuzwirbei-Segment (S4), das Beckensegment (S5), die Oberschenkelknochen (S6),
- als hauptsächliche Spannmuskeln zur Herstellung des Gleichgewichtes werden angesehen: die wichtigsten Agonisten, Antagonisten, sowohl lateral, als auch median. Diese Muskeln, die zur Teilnahme an der Herstellung des Gleichgewichtes hinzugezogen werden, werden vom klinischen Anwender in Abhängigkeit von der Stellung der Einheit aus Wirbelsäule und Becken ausgewählt.

4. Verfahren nach irgendeinem der Patentansprüche 1 bis 3, **dadurch gekennzeichnet, dass** das Individuum in eine Stellung stabilen Gleichgewichtes gebracht wird, die aufeinanderfolgenden Positionen der globalen Schwereachse (5) während einiger Sekunden mit einer Frequenz von mehreren Erfassungen pro Sekunde gespeichert werden, wobei die Gesamtheit der gespeicherten Positionen eine elliptische Positionswolke bildet, die Dispersionsellipse bestimmt wird, die die genannte Positionswolke einhüllt, und die Röntgenbilder ausgewählt werden, die einer globalen Schwereachse (5) entsprechen, deren Position in der Dispersionsellipse enthalten ist.

5. Verfahren nach Patentanspruch 4, **dadurch gekennzeichnet, dass** in Abhängigkeit von der Dispersionsellipse die Ungewissheit in der Bewertung der Rückstell- oder Stützkrafte durch Berechnung bestimmt wird.

6. Vorrichtung zur Bewertung der Gleichgewichtskräfte des Skelettes, umfassend:
- eine Röntgenquelle (1) und Haltemittel für eine Zielplatte (2), die auf Röntgenstrahlen empfindlich ist,
- eine Tragplatte (3) für das Individuum, geeignet, das Individuum (4) an festgelegter Stelle zwischen der Röntgenquelle und den Zielplatten-Haltemitteln (2) zu tragen und Eildpositionssignale der Horizontalposition (Og) der globalen Schwereachse (5) des Individuums (4) zu erzeugen,
- Mittel (20) zur Digitalisierung des Röntgenbildes des Individuums auf der Zielplatte und **dadurch** zur Erzeugung eines digitalisierten Röntgenbildes, das in einem geeigneten Speicher gespeichert wird,
- Mittel (21) zur Überlagerung des digitalisierten Röntgenbildes des Individuums mit dem digitalisierten Bild des Schlagschattens (Hg) der globalen Schwereachse (5) auf der Zielplatte (2),
- Mittel (22) zur Auswahl der gelenkig verbundenen Skelett-Segmente und ihrer Gelenke, sowie der Angriffspunkte der Gleichgewichts-Spannmuskeln, auf dem digitalisierten Röntgenbild,
- Rechenmittel (21) und ein Programm zur Berechnung der Stärke der Rückstellkraft, die von den Gleichgewichts-Spannmuskeln ausgeübt wird, aus der Tatsache, dass sie das Drehmoment der Schwerkraft, die an jedes Segment angreift, um das untere Gelenk des genannten Segmentes kompensiert.

7. Vorrichtung nach Patentanspruch 6, **dadurch gekennzeichnet, dass** sie Mittel zur Bewertung der Änderungen in der Vertikaistellung des Individuums durch die Abmessungen der Dispersionsellipse der horizontalen Position seiner globalen Schwereachse (5), wenn es eine stabile Stellung einnimmt, umfasst.

8. Vorrichtung nach einem der Patentansprüche 6 oder 7, **dadurch gekennzeichnet, dass** das Programm eine oder mehrere Erfassungssequenzen umfasst, in denen
- der Bediener auf dem Röntgenbild die Positionen der Gelenke der Segmente und der Angriffspunkte der Gleichgewichts-Spannmuskeln bezeichnet,
- die Mittel (20) zur Digitalisierung des Röntgenbildes des Individuums im Speicher die Positionen speichern, die vom Bediener bezeichnet wurden.

9. Vorrichtung nach einem der Patentansprüche 6 oder 7, **dadurch gekennzeichnet, dass** das Programm eine oder mehrere Erfassungssequenzen umfasst, in denen
- der Bediener das digitalisierte Röntgenbild auf einem Bildschirm (23) anzeigt,
- der Bediener auf dem digitalisierten Röntgenbild mit Hilfe einer Ein-/Ausgabevorrichtung der Art einer Tastatur (22) oder einer Maus die Positionen der Gelenke, der Segmente und der Angriffspunkte der Gleichgewichts-Spannmuskeln bezeichnet,
- das Programm die Koordinaten der Punkte speichert, die vom Bediener bezeichnet wurden.

10. Vorrichtung nach einem der Patentansprüche 8 oder 9, **dadurch gekennzeichnet, dass** das Programm die Abmessungen der Dispersionsellipse der Horizontalposition der globalen Schwereachse (5) berücksichtigt, um einen Bereich der Ungewissheit der Berechnung der Muskel- und Berührungskräfte zu berechnen.
